(19) Europäisches Patentamt European Patent Office Office européen des brevets

(11) EP 4 603 445 A1

## (12) EUROPEAN PATENT APPLICATION

(43) Date of publication:
20.08.2025 Bulletin 2025/34

(21) Application number: 24157805.3

(22) Date of filing: 15.02.2024

(51) International Patent Classification (IPC):
**B81B 7/00** (2006.01)

(52) Cooperative Patent Classification (CPC):
**B81B 7/0006; A61B 5/686; A61B 5/6868;
A61N 1/05; A61N 1/0529;** A61B 5/263; A61B 5/266;
A61B 5/291; A61B 5/294; A61B 2562/125;
B81B 2201/058; B81B 2201/06; B81B 2203/0338

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: Albert-Ludwigs-Universität Freiburg
79098 Freiburg (DE)

(72) Inventors:
• KIELE, Patrick
79106 Freiburg (DE)

• BASLAN, Yara
79110 Freiburg (DE)
• STIEGLITZ, Thomas
79110 Freiburg (DE)
• BIELEFELDT, Sira
70599 Stuttgart (DE)
• CVANCARA, Paul
79104 Freiburg (DE)

(74) Representative: Grünecker Patent- und
Rechtsanwälte
PartG mbB
Leopoldstraße 4
80802 München (DE)

## (54) ELECTRODE ARRANGEMENT AND FABRICATION METHOD BASED ON OSMOTIC EFFECTS

(57) The present disclosure relates to an electrode arrangement and a corresponding fabrication method. More generally, the present disclosure relates to the fabrication of electrically conductive channels using osmotic effects. An electrode arrangement (100; 200) comprises an electrically insulating channel layer (102), an electrically insulating first cover layer (106), an electrically insulating second cover layer (108), wherein at least one of the first and second cover layer (106, 108) is water-permeable, wherein the channel layer (102) comprises at least one connecting channel (104), the connecting channel (104) comprising a water-soluble component, and wherein the connecting channel (104) is connected to at least two water-insoluble contact units (110, 112) for electrically contacting the connecting channel (104).

Fig. 1

EP 4 603 445 A1

**Description**

The present disclosure relates to an electrode arrangement and a corresponding fabrication method. More generally, the present disclosure relates to the fabrication of electrically conductive channels using osmotic effects.

Implantable polydimethylsiloxane (PDMS)-based electrode arrays usually consist of a flexible PDMS substrate with an embedded electrically conductive material, usually a precious metal such as platinum or platinum iridium, which is responsible for contacting the electrodes and the electronics. The mechanical strength of the electrode arrays plays a significant role in their durability after implantation.

The mechanical stability of the electrode arrays is evaluated by means of bending and stretching tests, in which delamination and fracture damage to the conductor tracks occur. These failures depend on the large difference in the E-modules of metal and PDMS. Important fields of application for such electrode arrays can be seen in electrical stimulation of the brain, or in performing electrical measurements at the living organism.

Microelectromechanical systems (MEMS) are of high interest for recent electronic applications. Their applications range from medicine to measurement technology, from microfluidics to the Internet of Things (IoT). In many cases, MEMS elements serve as sensors or actuators, e.g., in recent mobile phones, but also in future autonomously driving cars. Most MEMS elements are based on silicon, which is not deformed plastically under a load, as opposed to metals. While highly sophisticated solutions were already found for diverse MEMS sensors, actuators, and other elements, MEMS fabrication is less standardized than pure microelectronics, which sometimes blocks new ideas. One of the possibilities to overcome this problem may be the 3D printing approach. While most 3D printing technologies do not offer sufficient resolution for MEMS production, and many of the common 3D printing materials cannot be used for this application, there are still niches in which the 3D printing of MEMS enables producing new structures and thus creating elements for new applications, or the faster and less expensive production of common systems. In the article Blachowicz, T., Ehrmann, A. 2020. 3D Printed MEMS Technology-Recent Developments and Applications. Micromachines 11, 4, an overview is given of the most recent developments and applications in 3D printing of MEMS.

Further, the article Cramer, S. W., Carter, R. E., Aronson, J. D., Kodandaramaiah, S. B., Ebner, T. J., and Chen, C. C. 2021. Through the looking glass: A review of cranial window technology for optical access to the brain. Journal of neuroscience methods 354, 109100, describes techniques for deciphering neurologic function by analyzing the neuronal networks and emergent properties that arise from the interactions among single neurons. Mechanistic insights into neuronal networks require tools that simultaneously assess both single neuron activity and the consequent mesoscale output. The development of cranial window technologies, in which the skull is thinned or replaced with a synthetic optical interface, has enabled monitoring neuronal activity from subcellular to mesoscale resolution in awake, behaving animals when coupled with advanced microscopy techniques. In this article, recent achievements in cranial window technologies are reviewed, appraising the relative merits of each design and discussing the future research in cranial window design.

The publication Park, D.-W., Brodnick, S. K., Ness, J. P., Atry, F., Krugner-Higby, L., Sandberg, A., Mikael, S., Richner, T. J., Novello, J., Kim, H., Baek, D.-H., Bong, J., Frye, S. T., Thongpang, S., Swanson, K. I., Lake, W., Pashaie, R., Williams, J. C., and Ma, Z. 2016. Fabrication and utility of a transparent graphene neural electrode array for electrophysiology, in vivo imaging, and optogenetics. Nature protocols 11, 11, 2201-2222, describes transparent graphene-based neural electrode arrays which are intended to provide opportunities for simultaneous investigation of electrophysiology, various neural imaging modalities, and optogenetics. Graphene electrodes have previously demonstrated greater broad-wavelength transmittance (-90%) than other transparent materials such as indium tin oxide (-80%) and ultrathin metals (-60%). This publication describes how to fabricate and implant a graphene-based microelectrocorticography (mECoG) electrode array and subsequently use this alongside electrophysiology, fluorescence microscopy, optical coherence tomography (OCT), and optogenetics. Further applications, such as transparent penetrating electrode arrays, multi-electrode electroretinography, and electromyography, are also viable with this technology.

Electrical stimulation using electrodes is widely used to treat various neuronal disorders such as Parkinson's disease and epilepsy and is a widely used research tool in neuroscience studies. However, devices that help better understand the mechanisms of electrical stimulation in neural tissues have been limited to opaque neural electrodes. Imaging spatiotemporal neural responses to electrical stimulation with minimal artifact could allow for various studies that are impossible with existing opaque electrodes. The article Park, D.-W., Ness, J. P., Brodnick, S. K., Esquibel, C., Novello, J., Atry, F., Baek, D.-H., Kim, H., Bong, J., Swanson, K. I., Suminski, A. J., Otto, K. J., Pashaie, R., Williams, J. C., and Ma, Z. 2018. Electrical Neural Stimulation and Simultaneous in Vivo Monitoring with Transparent Graphene Electrode Arrays Implanted in GCaMP6f Mice. ACS nano 12, 1, 148-157, describes electrical brain stimulation and simultaneous optical monitoring of the underlying neural tissues using carbon-based, fully transparent graphene electrodes implanted in GCaMP6f mice. Fluorescence imaging of neural activity for varying electrical stimulation parameters was conducted with minimal image artifact through transparent graphene electrodes. In addition, full-field imaging of electrical stimulation verified more efficient neural activation with cathode leading stimulation compared to anode leading stimulation. The charge density limitation of capacitive four-layer graphene electrodes has been characterized as 116.07-174.10 $\mu C/cm^2$ based on electrochemical impedance spectroscopy, cyclic voltammetry, failure bench testing, and in vivo testing. This study demonstrates the transparent ability of graphene neural electrodes and provides a method to further increase understanding and potentially improve therapeutic electrical stimulation in the central and peripheral nervous systems.

Further, US 2016/007874 A1 relates to transparent and flexible neural electrode arrays. Devices for detecting electrical activity in electrically active biological tissues and methods for using the devices are provided. The devices include an electrode array that is configured for implantation on electrically active biological tissue. The electrode array comprises a plurality of electrode sites comprising one or more layers of transparent, electrically conductive graphene disposed on a transparent substrate.

CN108744268A discloses an application of a flexible transparent carbon nano tube neural electrode array in a neural photoelectric interface. A flexible transparent carbon nano electrode array has a high light transmittance in a wide wavelength interval and can still maintain good electrochemical properties and optical properties in a stretching state to achieve simultaneous recording of cortex epilepsy electrophysiological signals and two-photon calcium imaging and real-time recording of brain electricity in the photostimulation so as to observe the nerve motion with high time/spatial resolution. The good flexibility and the extensibility of the carbon nano tube electrode array are employed and the flexible transparent carbon nano tube neural electrode array can be used for real-time monitoring of the brain electricity motion in the brain damaging; and moreover, the carbon nano tube neural electrode array can be perfectly fit with a tissue to form an efficient brain/electronic interface and facilitate improving of the signal to noise ratio. The results above all show the huge utilization potentiality of the carbon nano tube neural electrode array in a nervous system especially in a mechanical active system electro-optic interface.

US 2021/0244304 A1 A discloses a transparent flexible bio-electrode and a method for manufacturing the same. The transparent flexible bio-electrode includes a substrate, electrode sites disposed at one side on the substrate, electroconductive contacts disposed at the other side on the substrate, and an interconnector configured to connect the electrode sites and the contacts.

It has been found that the difference between the E-modules of metal and PDMS is too high for many applications because delamination and breakage of the conductor tracks occurs due to mechanical stress. Thus, material transitions are the cause of failure because the involved materials are not mechanically adapted.

There are limited options for applicable designs for the existing concept of electrodes and a customization to patients only possible to a limited extent. Alternative materials, e.g. PDMS with conductive filler material such as silver, are usually not biocompatible or certified for implantation.

Therefore, there is still a need for an electrode concept that provides mechanical flexibility, long-term stability and electrical reliability, and at the same time can be fabricated in a cost-effective manner and with the possibility to be customized easily. Furthermore, the electrodes should advantageously be usable as implanted electrodes.

This object is solved by the subject matter of the independent claims. Advantageous embodiment are the subject matter of the dependent claims.

The disclosure is intended to protect the concept of realizing a mechanically homogeneous implantable PDMS-based electrode array. The focus is on the utilization of the osmotic effect for the construction of a conductive interface in the PDMS substrate. The reliability and service life of the structure are improved compared to known electrode concepts.

In particular, the present disclosure provides an electrode arrangement comprising:

an electrically insulating channel layer,

an electrically insulating first cover layer,

an electrically insulating second cover layer,

wherein at least one of the first and second cover layer is water-permeable,

wherein the channel layer comprises at least one connecting channel, the connecting channel comprising a water-soluble component - which nonetheless has a low permeability to the surrounding polymer - and wherein the connecting channel comprises at least two water-insoluble contact units for electrically contacting the connecting channel.

Such a water-soluble component provides in operation an electrically conductive connecting channel when water enters the connecting channel through the first and/or second cover layer. An example for a material used in the first and/or second cover layer is, for instance, PDMS. This polymer has a semi-permeable property that allows the diffusion of water molecules, but no ion exchange, for example sodium or chloride ions from body fluids. During fabrication of the channel electrodes, a water-soluble solid is introduced into the polymer, to form a channel. After partial or complete dissolution, this material forms an electrical connection between the electrode contacts (the water-soluble component may for instance comprise NaCl or a hydrogel). The contacts seal the channel with an electrically conductive or dielectric material, which is not soluble in water and is corrosion-resistant (e.g. Pt-Ir).

When the electrode array is implanted or otherwise put into an aqueous medium, water vapor diffuses from the surrounding body fluid into the above-mentioned channel due to the diffusion force and the solid dissolves in the channel. In addition, an osmotic force is created due to the difference in concentration between the surrounding body fluid and the channel. This attracts further water molecules into the channel, which becomes electrically conductive and contacts the two end plates. The specific electrical conductivity of the connection channel depends on the kind of water-soluble component used in the connecting channel. The osmotic force continues to draw in water until a concentration equilibrium has been reached or a pressure equilibrium has been reached between the osmotic pressure and the back pressure of the closed polymer matrix. The osmotic pressure is described by van 't Hoff's law as will be explained in more detail below.

Electrical contact to the end plates can be ensured by the magnitude of the osmotic pressure in the channel. The end plates prevent ion exchange between the channel and the surrounding body tissue. At the same time, they serve as an electrical stimulation or discharge contact or for further assembly and connection techniques. The contacts may e.g. form soldered or welded connections to implanted cables.

Although in the following the electrode arrangement according to the present disclosure is mainly described in the application example of implanted electrodes, it is clear that the principle of a conductive channel which is created by an aqueous solution entering a connecting channel comprising a water-soluble component can be applied to a great number of other applications.

Advantageously, metallic conductive leads can be avoided and thus, the electrodes according to the present disclosure are not only bendable, but also expandable. Moreover, the absence of metal leads may improve the compatibility of the electrode arrangement for magnetic resonance imaging. Further, the above-mentioned E-module mismatch can be avoided, so that more stable electrodes without cracking are provided.

Furthermore, an interesting effect of the electrode arrangement according to the present disclosure could be observed under mechanical stress: If the conductive duct is constricted due to excessive bending or tensile stress, the conductive duct is self-healing and thus no remaining interruption of the electrical connection occurs.

Moreover, the electrode arrangement according to the present disclosure can be fabricated using the principles of rapid prototyping as for instance described in the above-mentioned article Blachowicz, T., Ehrmann, A. 2020. 3D Printed MEMS Technology-Recent Developments and Applications. Micromachines 11, 4.

Advantageously, the electrode arrangement according to the present disclosure allows for the fabrication of optically transparent electrode arrays, which can be used in the cranial window technique for simultaneous optical access to the brain and electrical stimulation/recording of brain signals described in the articles mentioned above.

By integrating radiation emitting units such as LEDs behind the conductive structures, there is the additional advantageous option to combine the electrode arrangement with optogenetic approaches. Optogenetic techniques have been developed to allow control over the activity of selected cells within a highly heterogeneous tissue, using a combination of genetic engineering and light. Optogenetics employs natural and engineered photoreceptors, mostly of microbial origin, to be genetically introduced into the cells of interest. As a result, cells that are naturally light-insensitive can be made photosensitive and addressable by illumination and precisely controllable in time and space. The selectivity of expression and subcellular targeting in the host is enabled by applying control elements such as promoters, enhancers and specific targeting sequences to the employed photoreceptor-encoding DNA.

Advantageously, the water-soluble component comprises a salt and/or a hydrogel. The salt or hydrogel forms electrically conductive ions in aqueous surroundings. Hydrogels are water-retaining, porous polymer networks, which have tunable electrical properties and can be designed to become ion-conducting.

Furthermore, in order to electrically contact the connecting channel in operation, the at least two water-insoluble contact units comprise an electrically conductive and/or a dielectric material. Thereby, the contacting may be ohmic and/or capacitive. Capacitive contacting has the advantage that the external electronic components, which contact the electrode arrangement, can be hermetically sealed and are thus protected against the humid environment, in which the electrode arrangement is operating.

For instance, at least one of the at least two water-insoluble contact units may comprise a Pt-Ir layer. Platinum iridium alloys are conventionally used for the fabrication of metal microelectrodes for electrical stimulation of nervous tissue and electrophysiological recordings. Pt-Ir alloys have an advantageous combination of mechanical and electrochemical properties for this application.

Additionally, platinum iridium alloys containing oxides of both metals can be electro-deposited onto the surface of microelectrodes.

According to a further advantageous example of the present disclosure, the electrode arrangement comprises an adhesion promoting layer, which is arranged between the channel layer and/or the second cover layer and at least a part of the at least two water-insoluble contact units. For instance, the adhesion promoting layer may comprise a multilayer stack comprising $WTi/Si_xO_y$ and/or $WTi/SiC$ and/or $WTi/Si_xN_y$. The adhesion promoting layer optimizes the adhesion between the electrode contact and the first cover layer (e.g. PDMS), the phase boundary is optionally functionalized by a suitable layer composite (e.g. PtIr/WTi/SiN/PDMS, PtIr/WTi/$Si_xO_y$/PDMS, or PtIr/WTi/SiC/PDMS), so that covalent bonds are formed and delamination at the phase boundary is prevented.

Exemplarily, the channel layer and/or the first cover layer and/or the second cover layer comprise polydimethylsiloxane, PDMS.

According to an advantageous example of the present disclosure, the water-soluble component in the connecting channel is chosen so that in operation an osmotic pressure within the connecting channel is higher than outside the electrode arrangement. This osmotic overpressure ensures a safe electric contact between the connecting channel and the contact units.

According to an advantageous example of the present disclosure, the water-soluble component in the connecting channel comprises NaCl. This choice of material ensures in the setting of an implantation that no physiologically critical or poisonous substances are introduced into the organism, in which the electrode arrangement is implanted.

In particular for the application in the context of cranial windows, it is advantageous if the electrode arrangement is transparent in at least a part of a region, in which the connecting channel is arranged.

Advantageously, the electrode arrangement may be implantable.

The present disclosure thus also relates to the use of an electrode arrangement according to one of the aspects given above as an implanted measurement and/or excitation electrode arrangement. Because of the various advantageous characteristics, the electrode arrangement according to the present disclosure is implantable without problems, combining optical transparency and mechanical flexibility. Furthermore, complex arrays of a plurality of electrodes and optionally additional components can be realized. The electrode arrangement may be kept on stock in a dry condition and only be put into an operating status by an automatic activation due to the inflowing water when being put into the operation environment, such as the organism, in which the electrode arrangement is implanted. Thus, a much longer shelf like can be achieved.

Furthermore, the present disclosure provides a method of fabricating an electrode arrangement, the method comprising the following steps:

providing an electrically insulating first cover layer;

depositing a water-soluble component for forming a connecting channel;

depositing an electrically insulating channel layer;

depositing at least two water-insoluble contact units for electrically contacting the connecting channel;

providing an electrically insulating second cover layer, wherein at least one of the first and second cover layers is water-permeable,

wherein the at least two water-insoluble contact units are accessible from outside the electrode arrangement.

According to a first advantageous example, the step of depositing a water-soluble component for forming a connecting channel may comprise 3D-printing a pre-cursor of the water-soluble component and subsequently curing the pre-cursor. The water-soluble component, which in operation is electrically conductive, is fabricated as a self-supported structure and is partly enclosed by the channel layer only in a next step.

According to another advantageous example, the channel layer may also comprise a recess, wherein the recess is filled with the water-soluble component for forming the connecting channel.

The accompanying drawings are incorporated into and form a part of the specification to illustrate several embodiments of the present disclosure. These drawings together with the description serve to explain the principles of the disclosure. The drawings are merely for the purpose of illustrating the preferred and alternative examples of how the disclosure can be made and used, and are not to be construed as limiting the disclosure to only the illustrated and described embodiments. Furthermore, several aspects of the embodiments may form-individually or in different combinations-solutions according to the present disclosure. Further features and advantages will become apparent from the following more particular description of the various embodiments of the disclosure, as illustrated in the accompanying drawings, in which like references refer to like elements, and wherein:

FIGURE 1      shows schematically a cross-sectional view of an electrode arrangement according to the present disclosure;

FIGURE 2A-2E   schematically illustrate the manufacturing steps for fabricating an electrode arrangement according to the present disclosure;

FIGURE 3      shows a schematic sectional view of a cranial window with an implanted cortical electrode array;

FIGURE 4      shows a schematic top view of a cortical electrode array using the electrode arrangement according the present disclosure.

The present disclosure will now be explained in more detail with reference to the Figures.

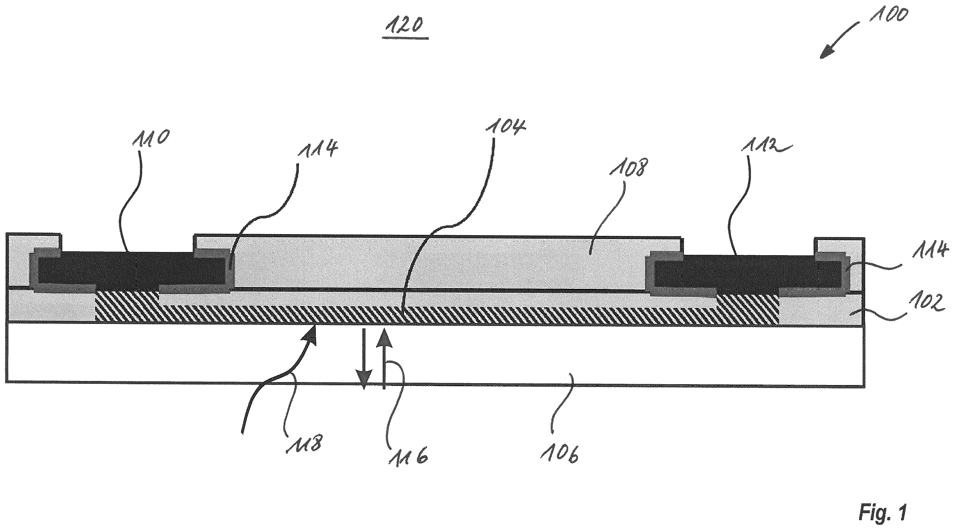

Referring now to Figure 1, an electrode arrangement 100 comprises a channel layer 102, in which a connecting channel 104 is arranged. The channel layer 102 comprises a water-soluble component. According to the present invention, if the water-soluble component comes into contact with water, the connecting channel 104 forms an electrically conductive lead. The channel layer 102 is covered by a first cover layer 106, and a second cover layer 108. According to the present disclosure, at least one of the first and second cover layers 106, 108, is water-permeable. Thus, when being brought into contact with aqueous surroundings, water diffuses through the water-permeable first and second cover layer 106, 108, and enters the connecting channel 104. Thereby, the water-soluble component is dissolved in water and thus provides ionic electrical conductivity. For instance, the water-soluble component may comprise sodium chloride.

The first cover layer 106 and/or the second cover layer 108 and/or the channel layer 102 are fabricated from a plastic material, such as polydimethylsiloxane (PDMS). As mentioned above, the PDMS material has semi-permeable characteristics. In other words, the diffusion of water molecules is possible, however, no exchange of ions is possible. During the fabrication a water-soluble solid material is introduced into the polymer matrix in order to form a channel. When being brought into contact with an aqueous solution, the water-soluble material is partly or fully dissolved by the inflowing water. For electrically contacting the connecting channel 104 from the outside, the connecting channel 104 is closed at both ends with a first and a second water-insoluble contact unit 110, 112.

The first water-insoluble contact unit 110 and the second water-insoluble contact unit 112 are either electrically conductive, so that they provide an ohmic electrical contact to the connecting channel 104, or they are made partly or fully from a dielectric material, so that a capacitive contact can be established. Of course, it is also possible to provide one or more ohmic contacts in combination with one or more capacitive contacts. Advantageously, the material of the water-insoluble contact units 110, 112 is corrosion resistant because it is in contact with the aqueous solution in which the electrode arrangement is operated. For instance, platinum-iridium alloys can be used for the outer surface of the water-insoluble contact units 110, 112. In case of capacitively contacting the contact units 110 and 112, an inert and corrosion-resistive dielectric material is chosen for the outer surface of the water-insoluble contact units 110, 112.

For enhancing the adhesion between the water-insoluble contact units 110, 112 and the surrounding plastic material, a suitable adhesion promoter 114 can be provided. For instance, the adhesion promoting layer 114 may comprise a multilayer stack comprising $WTi/Si_xO_y$ and/or $WTi/SiC$ and/or $WTi/Si_xN_y$. By using such an adhesion promoting layer 114, the phase boundary between the semipermeable material and the water-insoluble contact material is functionalized, so that covalent bonding is caused which prevents a delamination at the phase boundary.

When the electrode arrangement 100 is immersed into an aqueous environment, e.g. when it is implanted into an organism, water from the environment, e.g. the body fluid, diffuses due to the diffusion force 116 through the semi-permeable material (e.g. PDMS) of the first cover layer 106 and/or the second cover layer 108 and/or the channel layer 102 into the connecting channel 104.

Due to the entering water, the solid material at least partly dissolves in the channel. In addition, an osmotic force 118 is created due to the difference in concentration between the surrounding body fluid and the channel. This attracts further water molecules into the channel, which becomes electrically conductive and contacts the two water-insoluble contact units 110, 112, which may also be called end plates. The specific electrical conductivity kappa ($\kappa$) of an electrolyte is defined by

$$\kappa = \Lambda \cdot c$$

with

c = n/V    substance concentration (molar concentration) of the solution

$\Lambda$    molar limiting conductivity of the dissolved electrolyte

For the example of NaCl this becomes:

$$\kappa = \Lambda_{0,NaCl} \cdot c$$

with $\Lambda_{0,NaCl} = 126.9 \frac{S \cdot cm^2}{mol}$ being the molar limiting conductivity of dissolved NaCl.

The osmotic force 118 continues to draw in water until a concentration equilibrium has been reached or a pressure equilibrium has been reached between the osmotic pressure and the back pressure of the closed polymer matrix.

The osmotic pressure is described by van 't Hoff's law:

$$\Pi = i \cdot c \cdot R \cdot T$$

With:

| | |
|---|---|
| $\Pi$ | osmotic pressure |
| $c = n/V$ | substance concentration (molar concentration) of the solution |
| i | Van-'t-Hoff factor (i=2 for NaCl) |
| R | universal gas constant |
| T | absolute temperature in K |

Electrical contact to the end plates can be ensured by an osmotic overpressure in the channel. The end plates prevent ion exchange between the connecting channel 104 and the surrounding environment 120, e.g. body tissue. At the same time, the end plates 110, 112 serve as an electrical stimulation or discharge contact or for further assembly and connection techniques. The contacts may e.g. form soldered or welded connections to implanted cables.

Although in the following the electrode arrangement according to the present disclosure is mainly described in the application example of implanted electrodes, it is clear that the principle of a conductive channel, which is created by an aqueous solution entering a connecting channel comprising a water-soluble component can be applied to a great number of other applications.

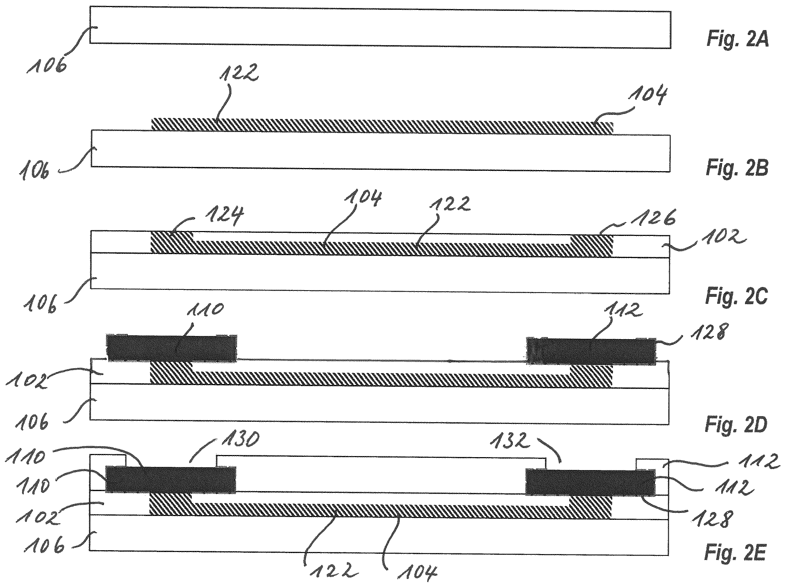

Figure 2 shows a sequence of fabrication steps for a manufacturing process according to a first example of the present disclosure.

According to an exemplary example, the process starts with providing the first cover layer 106 (Figure 2A). The first cover layer 106 is, for instance, fabricated from PDMS, which has a semi-permeable characteristics. In other words, PDMS allows the diffusion of water molecules, but does not allow an ion exchange.

Of course, the first cover layer 106 may also be fabricated from any other suitable semi-permeable polymer material.

In the next step (Figure 2B), the solid-state electrolyte, which is later forming the connecting channel 104, is deposited on the first cover layer 106, which forms a base material. The solid-state electrolyte 122 is, for instance, structured in a three-dimensional form, as shown in Figure 2C, so that an electrical contact can easily be added. For instance, the solid-state electrolyte 122 is deposited on the base material of the first cover layer 106 by means of a 3D printing process.

Further, the channel layer 102 is deposited on the solid-state electrolyte 122 and structured so that the contact regions 124,126 remain accessible.

As shown in Figure 2D, a first water-insoluble contact unit 110 and a second water-insoluble contact unit 112 are added in a next step. Thereby, the connecting channel 104 is completely closed and the contact regions 124,126 can be electrically contacted. The first and second water-insoluble contact units 110,112 form end plates, which can be contacted by external electronic devices and or can form excitation electrodes. The first and second water-insoluble contact units 110,112 may for instance be formed from a platinum-iridium alloy, in order to allow an ohmic contact.

However, one or both contact units 110,112 may also be formed at least partly by dielectric material, so that a capacitive contacting is possible.

In order to improve the adhesion of the polymer material of the first cover layer 106 and the channel layer 102 to the material of the water-insoluble contact units 110,112, the contact units 110,112 may at least partly be covered with an adhesion promoting layer 128. The adhesion promoting layer may be formed by a single layer or may comprise a multilayer stack comprising $WTi/Si_xO_y$ and/or $WTi/SiC$ and/or $WTi/Si_xN_y$.

In a final step, as shown in Figure 2E, the second cover layer 108 is added. The cover layer 108 is structured to have openings 130, 132, through which the first and second water-insoluble contact units 110, 112 are accessible. It should be noted, that the polymer material of the first and second cover layers 106, 108 and of the channel layer 102 may be identical or different. Furthermore, the first and second cover layers 106, 108 and the channel layer 102 may be deposited onto the solid state electrolyte 122 or may be prefabricated to have a channel structure.

The electrode arrangement 100, which is shown in Figure 2E, can be stored in its dry state and has a rather long shelf life. Only when the dry electrode arrangement 100 is introduced into an aqueous environment, water starts permeating through the first cover layer 106 and/or the second cover layer 108 and into the connecting channel 104. The materials of the first cover layer 106, the second cover layer 108 and the channel layer 102 have a high permeability for water vapor and a limited permeability for ions. In particular, the ions present in the solid-state electrolyte 122 advantageously cannot permeate through the first and second cover layers 106, 108 and the channel layer 102. Water, which enters into the connecting channel 104 and condenses therein, dissolves the solid-state electrolyte 122. Due to osmosis, further water is drawn into the connecting channel 104. A stable equilibrium is achieved by the osmotic pressure in equilibrium with the counter pressure of the polymer. In this state, the electrolyte is dissolved in liquid and leads to an electric conductivity.

As an alternative to solid state electrolytes, also hydrogels with ions held therein can be used for forming the connecting channel 104. As mentioned above, also further components may be integrated into the electrode arrangement 100. For instance, LEDs may be added in order to allow these electrode arrangements to be used for optogenetic techniques.

Advantageously, the electrode arrangement 100 is transparent in all those regions that are not covered by metallic contact units 110, 112. Thus, the electrode arrangement may be used to form leads for use in cranial window technology, allowing simultaneous optical access to the brains and electrical stimulation and monitoring of brain signals. As mentioned above, the fabrication of the electrodes can be achieved using the principles of rapid prototyping. In case the conductive connecting channel 104 is constricted due to excessive bending or tensile strain, the connecting channel 104 is self-healing. The pressure equilibrium works to reinstate the previous situation. As no metallic long leads are involved, the electrode arrangement 100 is not only bendable, but also stretchable. Further, the electrode arrangement 100 is much more resistive against damaging and cracks forming due to bending compared to metallic electrodes.

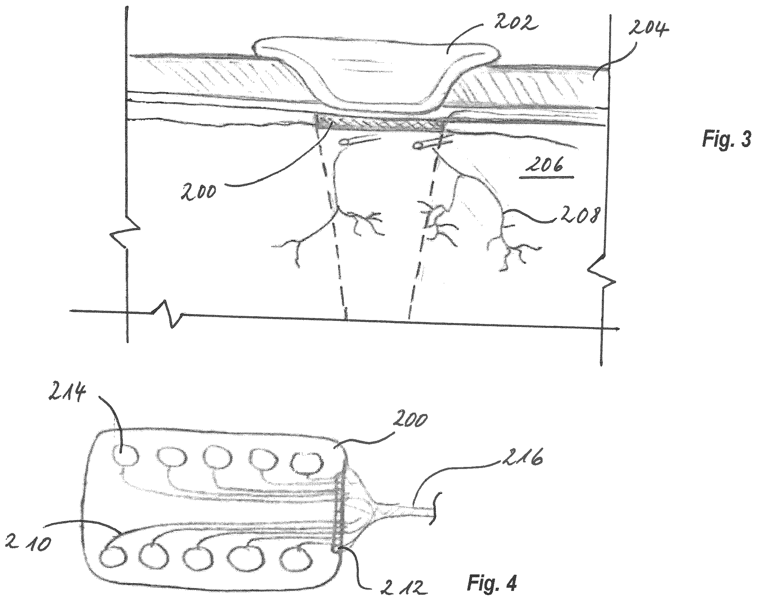

Figure 3 illustrates an example, where the electrode arrangement according to the present disclosure forms a cortical electrode array which is implanted on the cerebral cortex below a cranial window 202. The cranial window 202 is arranged in an opening within the cranial bone 204. The electrode arrangement 200 is in contact with the brain tissue 206 and the blood vessels 208. After implantation, the water contained in the brain tissue 206 permeates into the previously dry connecting channel and establishes a conductive lead as explained above regarding the electrode arrangement 100 of Figures 1 and 2.

The cranial window 202 allows an optical access to the brain tissue 206 and blood vessels 208 situated below (as indicated by the broken lines). Due to the transparency of the electrode arrangement 200 the optical access is not inhibited by the presence of the electrode arrangement 200. Integrating the electrode arrangement 200 below the cranial window 202 allows the simultaneous acquisition of electrical signals and optical images.

Figure 4 shows a top view of the cortical electrode array 200 usable for the cranial window of Figure 3. The cortical electrode array 200 has a plurality of connecting channels 210, which connect an input/output connector 212 to a plurality of electrode plates 214. The connecting channels 210 are formed as connecting channels 102 described above with reference to Figures 1 and 2 and the electrode plates 214 represent water-insoluble contact units according to the present disclosure. The connecting channels 210 form an optically transparent area through the complete cross-section of the cortical electrode array 200.

An electrical connection e.g. to a cable 216 is established via the input/output connector 212. The input/output connector 212 comprises water-insoluble contact units as explained above with reference to Figure 1 and 2. In particular, the electrical contact can be made by ohmic connection or capacitive contact.

In conclusion, this disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

REFERENCE NUMERALS

| Reference Numeral | Description |
|---|---|
| 100 | electrode arrangement |
| 102 | channel layer |
| 104 | connecting channel |
| 106 | first cover layer |
| 108 | second cover layer |
| 110 | first water-insoluble contact unit; end plate |
| 112 | second water-insoluble contact unit; end plate |
| 114 | adhesion promoting layer |
| 116 | diffusion force |
| 118 | osmotic force |

(continued)

| Reference Numeral | Description |
|---|---|
| 120 | surrounding environment |
| 122 | solid state electrolyte |
| 124 | first contact region |
| 126 | second contact region |
| 128 | adhesion promoting layer |
| 130 | first opening |
| 132 | second opening |
| 200 | electrode arrangement; cortical electrode array |
| 202 | cranial window |
| 204 | cranial bone |
| 206 | brain tissue |
| 208 | blood vessel |
| 210 | connecting channel |
| 212 | input/output connector |
| 214 | electrode plates |
| 216 | cable |

**Claims**

1. Electrode arrangement (100; 200) comprising:

   an electrically insulating channel layer (102),
   an electrically insulating first cover layer (106),
   an electrically insulating second cover layer (108),
   wherein at least one of the first and second cover layer (106, 108) is water-permeable,
   wherein the channel layer (102) comprises at least one connecting channel (104), the connecting channel (104) comprising a water-soluble component, and wherein the connecting channel (104) is connected to at least two water-insoluble contact units (110, 112) for electrically contacting the connecting channel (104).

2. Electrode arrangement according to claim 1, wherein the water-soluble component comprises a salt and/or a hydrogel.

3. Electrode arrangement according to claim 1 or 3, wherein the at least two water-insoluble contact units (110, 112) comprise an electrically conductive and/or a dielectric material.

4. Electrode arrangement according to claim 3, wherein at least one of the at least two water-insoluble contact units (110, 112) comprises a Pt-Ir layer.

5. Electrode arrangement according to one of the preceding claims, further comprising an adhesion promoting layer (114), which is arranged between the channel layer (102) and/or the second cover layer (108) and at least a part of the at least two water-insoluble contact units (110, 112).

6. Electrode arrangement according to claim 5, wherein the adhesion promoting layer (114) comprises a multilayer stack comprising $WTi/Si_xO_y$ and/or $WTi/SiC$ and/or $WTi/Si_xN_y$.

7. Electrode arrangement according to one of the preceding claims, wherein the channel layer (102) and/or the first cover layer (106) and/or the second cover (108) layer comprise polydimethylsiloxane, PDMS, and/or wherein the water-soluble component in the connecting channel (104) is chosen so that in operation an osmotic pressure within the connecting channel is higher than outside the electrode arrangement (100; 200).

8. Electrode arrangement according to one of the preceding claims, wherein the water-soluble component in the connecting channel (104) comprises NaCl.

9. Electrode arrangement according to one of the preceding claims, wherein the electrode arrangement (100; 200) is transparent in at least a part of a region, in which the connecting channel (104) is arranged.

10. Use of an electrode arrangement (100; 200) according to one of the preceding claims as an implanted measurement and/or excitation electrode arrangement.

11. Method of fabricating an electrode arrangement, the method comprising the following steps:

providing an electrically insulating first cover layer (106);
depositing a water-soluble component for forming a conductive connecting channel (104);
depositing an electrically insulating channel layer (102);
depositing at least two water-insoluble contact units (110, 112) for electrically contacting the connecting channel (104);
providing an electrically insulating second cover layer (108), wherein at least one of the first and second cover layers (106, 108) is water-permeable,
wherein the at least two water-insoluble contact units (110, 112) are accessible from outside the electrode arrangement (100; 200).

12. Method according to claim 11, wherein the step of depositing a water-soluble component for forming a connecting channel (104) comprises 3D-printing a pre-cursor of the water-soluble component and subsequently curing the pre-cursor.

13. Method according to claim 11 or 12, further comprising the step of coating the water-insoluble contact units (110, 112) at least partly with an adhesion promoting layer (114).

14. Method according to claim 13, wherein the adhesion promoting layer (114) comprises a multilayer stack comprising $WTi/Si_xO_y$ and/or $WTi/SiC$ and/or $WTi/Si_xN_y$.

15. Method according to one of the claims 11 to 14, wherein the channel layer (102) comprises a recess and wherein the recess is filled with the water-soluble component for forming the connecting channel (104).

Fig. 1

Fig. 2A

Fig. 2B

Fig. 2C

Fig. 2D

Fig. 2E

EP 4 603 445 A1

Fig. 3

Fig. 4

EP 4 603 445 A1

Europäisches
Patentamt

European
Patent Office

Office européen
des brevets

# EUROPEAN SEARCH REPORT

**Application Number**

EP 24 15 7805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/324107 A1 (NISHIZAWA MATSUHIKO [JP] ET AL) 15 October 2020 (2020-10-15) | 1-7,9-15 | INV. B81B7/00 |
| Y | * figures 8A-8D * | 8 | |
| X | NISHIMURA AYAKA ET AL: "Totally transparent hydrogel-based subdural electrode with patterned salt bridge", BIOMEDICAL MICRODEVICES, SPRINGER US, NEW YORK, vol. 22, no. 3, 22 August 2020 (2020-08-22), XP037224988, ISSN: 1387-2176, DOI: 10.1007/S10544-020-00517-0 [retrieved on 2020-08-22] | 1,11 | |
| Y | * figures 1,2 * | 8 | |
| A | WO 2019/047282 A1 (UNIV ZHEJIANG [CN]) 14 March 2019 (2019-03-14) * figure 1 * | 1-15 | |

**TECHNICAL FIELDS SEARCHED (IPC)**

B81B

The present search report has been drawn up for all claims

| Place of search | Date of completion of the search | Examiner |
|---|---|---|
| Berlin | 24 June 2024 | McGinley, Colm |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 15 7805

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2024

| Patent document cited in search report | Publication date | Patent family member(s) | Publication date |
|---|---|---|---|
| US 2020324107 A1 | 15-10-2020 | JP 7228903 B2 | 27-02-2023 |
| | | JP WO2019124566 A1 | 25-02-2021 |
| | | US 2020324107 A1 | 15-10-2020 |
| | | WO 2019124566 A1 | 27-06-2019 |
| WO 2019047282 A1 | 14-03-2019 | CN 107638176 A | 30-01-2018 |
| | | WO 2019047282 A1 | 14-03-2019 |

EPO FORM P0459

For more details about this annex : see Official Journal of the European Patent Office, No. 12/82

## REFERENCES CITED IN THE DESCRIPTION

### Patent documents cited in the description

- US 2016007874 A1
- CN 108744268 A

- US 20210244304 A1

### Non-patent literature cited in the description

- **BLACHOWICZ, T.** ; **EHRMANN, A.** 3D Printed MEMS Technology-Recent Developments and Applications.. *Micromachines*, 2020, vol. 11, 4
- **CRAMER, S. W.** ; **CARTER, R. E.** ; **ARONSON, J. D.** ; **KODANDARAMAIAH, S. B.** ; **EBNER, T. J.** ; **CHEN, C. C.** Through the looking glass: A review of cranial window technology for optical access to the brain.. *Journal of neuroscience methods*, 2021, vol. 354, 109100

- **PARK, D.-W.** ; **BRODNICK, S. K.** ; **NESS, J. P.** ; **ATRY, F.** ; **KRUGNER-HIGBY, L.** ; **SANDBERG, A.** ; **MIKAEL, S.** ; **RICHNER, T. J.** ; **NOVELLO, J.** ; **KIM, H.** Fabrication and utility of a transparent graphene neural electrode array for electrophysiology, in vivo imaging, and optogenetics.. *Nature protocols*, 2016, vol. 11 (11), 2201-2222
- **PARK, D.-W.** ; **NESS, J. P.** ; **BRODNICK, S. K.** ; **ESQUIBEL, C.** ; **NOVELLO, J.** ; **ATRY, F.** ; **BAEK, D.-H.** ; **KIM, H.** ; **BONG, J.** ; **SWANSON, K. I.** Electrical Neural Stimulation and Simultaneous in Vivo Monitoring with Transparent Graphene Electrode Arrays Implanted in GCaMP6f Mice.. *ACS nano*, 2018, vol. 12 (1), 148-157